# DEMANDE DE BREVET EUROPEEN

(11) **EP 4 821 433 A1**
(43) Date de publication de la demande: **14.10.2026**
(21) Numéro de dépôt: 26169459.0
(22) Date de dépôt: 31.03.2026
(51) Int. Cl.: A61B 5/145, A61B 5/00, A61M 37/00, A61N 1/30

(54) **SYSTÈME DE DÉLIVRANCE/EXTRACTION DE FLUIDE À TRAVERS LA PEAU D'UN ÊTRE VIVANT**

(30) Priorité: 10.04.2025 FR 2503845
(71) Demandeur: COMMISSARIAT À L'ÉNERGIE ATOMIQUE ET AUX ÉNERGIES ALTERNATIVES, 75015 Paris (FR)
(72) Inventeur: REVAUX, Amélie, 38054 Grenoble Cedex 09 (FR); PHAM, Pascale, 38054 Grenoble Cedex 09 (FR); THOMAS, Yohann, 38054 Grenoble Cedex 09 (FR)
(74) Mandataire: INNOV-GROUP

(57) **Abrégé**

L'invention concerne un dispositif adapté pour être employé dans un système de délivrance/extraction de fluide à travers la peau d'un être vivant, ledit dispositif comprenant :
- Un support (2) portant N microaiguilles, chaque microaiguille (3) comportant un corps (30) dans lequel est réalisé un canal (31) de délivrance/extraction de fluide (F), par lequel le fluide est apte à s'écouler lors de sa délivrance ou de son extraction,
- Le dispositif comportant une première zone conductrice (A) réalisée sur la face interne (20) du support et une deuxième zone conductrice (B) réalisée sur la face externe (21) du support,
- Une troisième zone conductrice (C) étant réalisée sur la paroi interne (310) du canal (31) de chaque microaiguille pour relier la première zone conductrice (A) à la deuxième zone conductrice (B), la troisième zone conductrice (C) présentant une conductivité électrique inférieure à celle de la première zone conductrice et de la deuxième zone conductrice.

## Description

### Domaine technique de l'invention

La présente invention se rapporte à un système de délivrance/extraction de fluide à travers la peau d'un être vivant.

### Etat de la technique

Le fluide interstitiel (appelé couramment ISF) est le fluide qui occupe l'espace entre les capillaires sanguins et les cellules biologiques. C'est dans ce fluide que les cellules puisent leur nourriture, leur oxygène et facilitent les échanges de nutriments et de déchets. L'ISF est constitué principalement d'eau, sels, sucres, hormones, neurotransmetteurs, et d'acides gras. L'ISF représente une matrice de surveillance prometteuse car il peut être prélevé de manière très peu invasive à partir de la peau, en continu, sans avoir besoin des méthodes d'extraction plus invasives et douloureuses utilisées pour le sang. La surveillance en continu de l'ISF est très intéressante pour différentes applications, notamment pour réaliser un suivi du cortisol (marqueur de stress) et des hormones sexuelles, ainsi que pour suivre des patients en néphrologie, cardiologie ou oncologie. Dans le cas du monitoring en glucose pour les diabétiques, il est notamment possible de placer un capteur sous la peau, soit sur une microaiguille, soit sur un cathéter sous-cutané. Cependant, cela peut poser un problème de biocompatibilité et nécessiter une réelle miniaturisation des capteurs.

L'ISF est certes présent en quantité acceptable sous la peau (120 µL.cm⁻²) mais ne se renouvelle qu'avec un débit très faible, de l'ordre de 60 nL.min⁻¹.cm⁻². Son exploitation dans le cadre des applications précitées reste donc très compliquée.

Il existe plusieurs stratégies pour accéder à l'ISF :
- L'ionophorèse et l'électro-osmose sont utilisées pour récupérer l'ISF à travers la peau. L'ionophorèse est basée sur l'utilisation d'un signal électrique continu pour déplacer un fluide tandis que l'électro-osmose permet le mouvement d'un fluide adjacent à une surface plane et chargée sous l'influence d'un champ électrique appliqué parallèlement à la surface.
- Une autre stratégie consiste à réaliser une extraction de l'ISF par microaiguilles (creuses pour créer un canal, ou en hydrogel qui agit comme une éponge) et de réaliser une mesure déportée.

Le brevet EP1183065B1 et la demande de brevet US2007/078376A1 décrivent des solutions de délivrance/extraction de fluide à travers la peau d'un être vivant.

Les solutions présentées dans ces deux documents consistent à utiliser deux patchs à appliquer contre la peau, chaque patch comprenant une matrice de microaiguilles et formant chacun une électrode distincte. On vient ensuite appliquer une différence de potentiel entre les deux patchs pour forcer le déplacement de fluide par électro-osmose ou ionophorèse entre les deux patchs.

On constate que ces solutions ne sont pas adaptées à la délivrance/extraction de fluide (comme l'ISF) à travers la peau pour plusieurs raisons :
- Elles nécessitent l'utilisation de deux patchs séparés ;
- Elles sont particulièrement encombrantes ;
- La disposition des patchs doit être bien étudiée pour obtenir un déplacement de fluide suffisant ;

Le but de l'invention est de proposer un dispositif de délivrance/extraction de fluide (par exemple l'ISF) à travers la peau d'un être vivant, qui soit adapté pour résoudre les inconvénients des dispositifs antérieurs.

### Exposé de l'invention

Ce but est atteint par un dispositif adapté pour être employé dans un système de délivrance/extraction de fluide à travers la peau d'un être vivant, ledit dispositif comprenant :
- Un support comportant deux faces opposées, dites face interne et face externe, sa face interne comportant N microaiguilles, N étant supérieur ou égal à 1, chaque microaiguille comportant un corps dans lequel est réalisé un canal de délivrance/extraction de fluide, ce canal comportant une paroi interne le long de laquelle le fluide est apte à s'écouler lors de sa délivrance ou de son extraction,
- Le dispositif comportant une première zone conductrice réalisée sur la face interne du support et une deuxième zone conductrice réalisée sur la face externe du support et, pour chaque microaiguille :
- Une troisième zone conductrice est réalisée sur la paroi interne de son canal reliant la première zone conductrice à la deuxième zone conductrice, la troisième zone conductrice présentant une conductivité électrique inférieure à celle de la première zone conductrice et de la deuxième zone conductrice.

Selon une réalisation particulière, la première zone conductrice et la deuxième zone conductrice sont chacune réalisées respectivement par un premier dépôt conducteur et un deuxième dépôt conducteur.

Selon une autre réalisation particulière, le premier dépôt conducteur et le deuxième dépôt conducteur sont métalliques.

Selon une autre réalisation particulière, la troisième zone conductrice est réalisée par un troisième dépôt conducteur.

Selon une autre réalisation particulière, le troisième dépôt conducteur est réalisé sous la forme d'un polymère conducteur, ou d'un revêtement de type DLC.

Selon une autre réalisation particulière, la première zone conductrice se prolonge sur la face externe du support, en transitant par la tranche du support.

Selon une autre réalisation particulière, le dispositif comporte un dépôt d'un matériau isolant et biocompatible réalisé sur la première zone conductrice.

Selon une autre réalisation particulière, le corps de chaque micro-aiguille est réalisé dans un matériau choisi parmi le PLA (acide polylactique), le PLGA (acide polylactique-co-glycolique) et le verre.

Selon une autre réalisation particulière, le corps de chaque micro-aiguille et le support sont réalisés dans un même matériau.

L'invention concerne également un système de délivrance/extraction de fluide à travers la peau d'un être vivant, comprenant :
- Un dispositif destiné à être appliqué à travers la peau de l'être vivant,
- Une source d'alimentation électrique apte à imposer un signal électrique non continu,
Le dispositif étant tel que défini ci-dessus, la source d'alimentation électrique étant connectée à sa première zone conductrice et à sa deuxième zone conductrice.

Selon une réalisation particulière la source d'alimentation électrique est contrôlée pour imposer un signal électrique non continu.

Selon une autre réalisation particulière le signal électrique est un signal de tension ou un signal de courant imposé entre les deux zones conductrices.

Selon une autre réalisation particulière, le système comporte des moyens de réglage du débit de délivrance ou d'extraction du fluide, par ajustement en amplitude et en fréquence du signal électrique.

Contrairement à ce qui est proposé dans l'état de la technique où les forces électroosmotiques sont utilisées pour la délivrance transcutanée de principes actifs ou le prélèvement d'ISF à travers la barrière cutanée (avec ou sans création de pores cutanés au préalables), l'invention consiste à utiliser le canal de la microaiguille pour la gestion du flux fluidique à travers la peau.

### Brève description des figures

D'autres caractéristiques et avantages vont apparaître dans la description détaillée qui suit faite en regard des dessins annexés dans lesquels :
- La figure 1 représente un exemple de réalisation du système conforme à l'invention ;
- Les figures 2A à 2C montrent en perspective plusieurs architectures de réalisation d'une microaiguille employée dans le système de l'invention ;
- La figure 3 montre, vue en coupe axiale transversale, une réalisation avantageuse d'une microaiguille employée dans le dispositif de l'invention ;
- La figure 4 montre un tableau listant les différentes options envisageables pour la réalisation du dispositif ;
- Les figures 5A et 5B illustrent le principe de fonctionnement de l'invention ;

### Description détaillée d'au moins un mode de réalisation

Dans la suite de la description, le terme « conducteur » ou « conductrice » est employé au sens électrique, c'est-à-dire qu'il désigne un matériau capable de faire passer un courant électrique continu.

Le système de l'invention est destiné à la délivrance/extraction d'un fluide à travers la peau d'un être vivant. De manière non limitative, le fluide peut être par exemple le fluide interstitiel (ISF) évoqué ci-dessus.

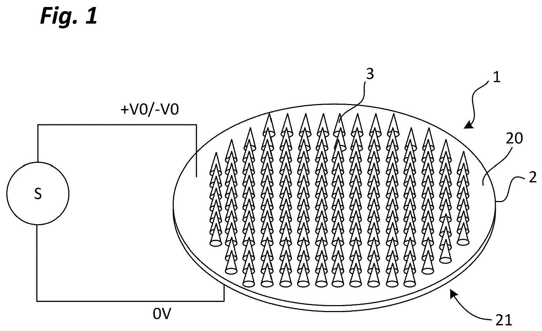

En référence à la figure 1, le système se compose d'un dispositif 1 à appliquer contre la peau de l'être vivant, adapté pour transpercer la peau de l'être vivant, et d'une source d'alimentation électrique S contrôlée pour imposer un signal électrique (tension électrique ou courant électrique) sur laquelle le dispositif 1 est connecté.

Le dispositif se présente sous la forme d'un unique patch et comporte un support 2. Le support 2 comporte deux faces opposées, dites face interne 20 destinée à être orientée en vis-à-vis de la peau à transpercer et une face dite externe 21 opposée, orientée vers l'extérieur. De manière non limitative, le support 2 peut par exemple présenter une forme de disque.

La face interne du support porte N microaiguilles 3 dressées, N étant supérieur ou égal à 1. Avantageusement, le support porte un nombre N de microaiguilles 3 qui est supérieur ou égal à 2, et même supérieur ou égal à 10. Dans la suite de la description, on considère un dispositif 1 doté de plusieurs microaiguilles 3.

Les microaiguilles 3 sont alignées et se dressent sur la face interne 20 du support, par exemple suivant plusieurs lignes et colonnes.

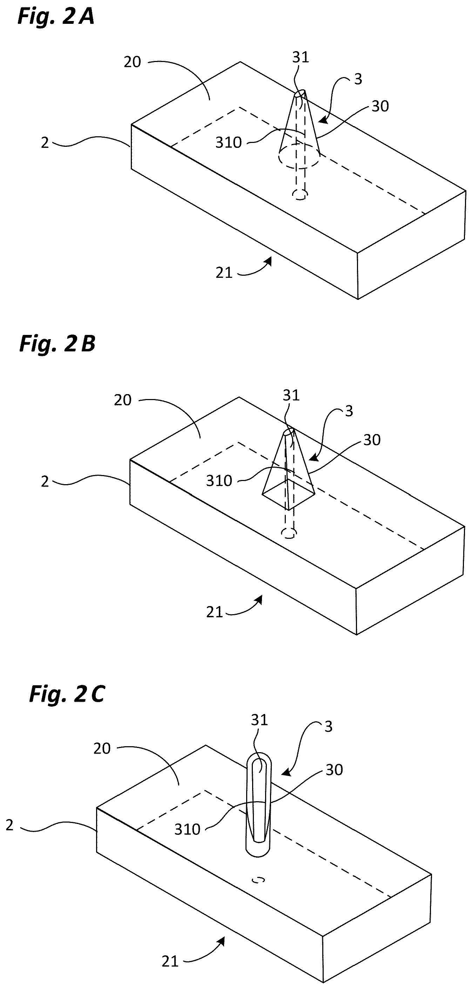

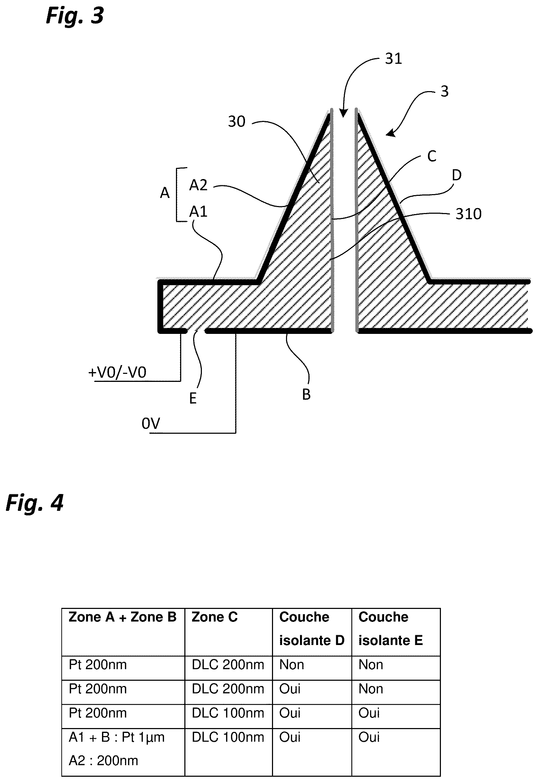

Sur la figure 1, il faut comprendre que les microaiguilles sont toutes creuses, par exemple selon l'une des architectures décrites ci-dessous en liaison avec les figures 2A à 2C et avec une configuration selon la figure 3.

En référence à la figure 2A, figure 2B et figure 2C, les microaiguilles 3 présentent chacune un corps 30. Les corps 30 des micro-aiguilles ont avantageusement tous une forme identique. Ce corps 30 comporte une paroi dite latérale formant le(s) flanc(s) de la microaiguille.

Les micro-aiguilles peuvent être réalisées en forme de cône (figure 2A), de pyramide (figure 2B), en forme de gouttière (figure 2C), et peuvent éventuellement être tronquées à leur extrémité finale, la troncature pouvant être réalisée en biseau.

Selon un aspect particulier de l'invention, les microaiguilles 3 comportent chacune un corps creux. Autrement dit, leur corps 30 comporte chacun un canal 31 transversal débouchant d'un côté à la pointe de la microaiguille 3 et de l'autre côté sur la face externe 21 du support. Différentes techniques de fabrication peuvent être envisagées pour la fabrication du corps creux de chaque microaiguille, par exemple moulage, microusinage...

Le canal 31 de chaque microaiguille 3 comporte une paroi interne 310, le long de laquelle le fluide sera amené à s'écouler dans le sens de la délivrance ou dans le sens de l'extraction. Ce canal 31 peut présenter une section constante ou non (par exemple évasée).

Il faut noter que la face interne 20 du support 2 inclut les flancs du corps de chaque microaiguille 3 qui se dresse sur cette face.

Le support 2 et les microaiguilles 3 peuvent être fabriquées à partir d'un même matériau. De manière non limitative, les microaiguilles 3 et le support 2 sont fabriquées à partir d'un matériau biopolymère ou d'un mélange de matériaux biopolymères. On a en effet constaté que de tels matériaux n'induisaient a priori aucune perturbation mesurable des biomarqueurs sériques d'infection, d'inflammation ou d'immunité. Les bioplastiques tels que le PLA (acide polylactique) et le PLGA (acide polylactique-co-glycolique) peuvent par exemple être choisis car déjà approuvés par les autorités réglementaires pour la conception d'implants médicaux, ceux-ci disposant en outre des propriétés mécaniques requises pour la perforation de la peau.

Selon un aspect particulier de l'invention, pour réaliser le prélèvement ou l'extraction du fluide à travers la peau de l'être vivant à l'aide du dispositif 1 à microaiguilles, on utilise le principe d'électro-osmose. Du point de vue de la physique, l'électro-osmose désigne la mise en mouvement d'un fluide contenant des charges libres (fluides biologiques) via l'application d'un champ électrique. Elle consiste plus précisément à mettre en contact le fluide biologique avec des électrodes, par exemple des électrodes micrométriques. Selon l'invention, l'écoulement de fluide, dans le sens de la délivrance ou dans le sens de l'extraction, est réalisé à travers le canal 31 de chaque microaiguille 3.

Pour mettre en œuvre le principe d'électro-osmose, le système comporte :
- Une première zone conductrice A réalisée sur la face interne du support (incluant les flancs du corps de chaque microaiguille-hors canal interne),
- Une deuxième zone conductrice B réalisée sur la face externe du support,
- Une troisième zone conductrice C réalisée sur la paroi interne 310 du canal de chaque microaiguille et réalisant une jonction conductrice entre la première zone conductrice A et la deuxième zone conductrice B.

Chaque zone conductrice est avantageusement matérialisée par au moins un dépôt conducteur réalisé sur le support 2.

Il faut noter que la troisième zone conductrice C s'applique au canal interne de chaque microaiguille des N microaiguilles.

La première zone conductrice A peut être matérialisée par deux dépôts conducteurs A1, A2 distincts avec des matériaux différents, avec un premier dépôt conducteur A1 situé sur la face interne en périphérie du corps de chaque microaiguille et un deuxième dépôt conducteur A2 situé sur le flanc de chaque microaiguille.

La première zone conductrice A peut se prolonger sur la face externe du support, via la tranche du support, permettant d'effectuer les deux reprises de contact sur la même face (externe) du support 2.

Bien entendu, au niveau de la reprise de contact, les deux zones conductrices A, B sont isolées électriquement l'une de l'autre (comme visible sur la figure 3 avec la zone E - voir ci-après).

Un dépôt D d'un matériau isolant et biocompatible peut être réalisé sur la face interne du support, pour recouvrir la première zone conductrice A.

Un matériau isolant identique (non représenté) est avantageusement également déposé sur la face externe du support pour recouvrir la deuxième zone conductrice B.

Selon un aspect particulier de l'invention, la troisième zone conductrice C présente une conductivité électrique inférieure à celle de la première zone conductrice A et de la deuxième zone conductrice B, permettant de générer une caractéristique résistive lorsqu'un signal électrique est appliqué entre la première zone conductrice A et la deuxième zone conductrice B.

Pour obtenir cette caractéristique résistive, plusieurs solutions sont envisageables, prises seules ou en combinaison :
- Jouer sur le type de matériau utilisé pour la troisième zone conductrice C, celui-ci étant par exemple choisi avec une conductivité électrique inférieure à celle du matériau utilisé pour la première zone conductrice A et la deuxième zone conductrice B ;
- Jouer sur l'épaisseur de la troisième zone conductrice C réalisée sur la paroi interne 310 du canal ;

Pour chaque zone conductrice A, B, C, on peut ainsi avoir les options et caractéristiques suivantes :

### Zone A + Zone B

Matériau bon conducteur (Or, Platine, ...) par exemple identique pour les deux zones.
Conductivité (désignée C_X) forte C_ZA=C_ZB>C_ZC

### Zone C :

Matériau faiblement conducteur (carbone, DLC, polymère conducteur, ...).
Conductivité C_ZC < C_ZA et C_ZC < C_ZB

A noter que la première zone conductrice A peut être scindée en deux dépôts conducteurs jointifs, A1, A2, utilisant des matériaux distincts (voir ci-après).

De manière non limitative, le tableau de la figure 4 indique plusieurs combinaisons et épaisseurs envisageables, pour chacune des zones conductrices du dispositif.

Ainsi, selon une configuration avantageuse, le support qui porte plusieurs microaiguilles 3 est recouvert sur toute la surface de sa face interne 20 (incluant les flancs de chaque microaiguille) d'un premier dépôt conducteur (zone conductrice A) et sur toute la surface de sa face externe 21 d'un deuxième dépôt conducteur (zone conductrice B). La zone conductrice C réalisée sous la forme d'un dépôt conducteur réalisé sur toute la paroi du canal interne de chaque microaiguille 3 effectue la jonction (mécanique et électrique) entre les zones conductrices A et B ainsi réalisées.

Le système comporte la source d'alimentation électrique S comprenant deux bornes sur lesquelles on vient respectivement connecter la première zone conductrice A et la deuxième zone conductrice B. La source d'alimentation électrique S est contrôlée pour imposer un signal électrique (courant électrique ou tension électrique) entre les deux zones conductrices.

Connectées à la source d'alimentation électrique S, les deux zone conductrices A, B sont ainsi placées chacune à un potentiel électrique distinct. De manière non limitative, l'une des zones conductrices est mise à un potentiel non nul +V0/-V0 tandis que l'autre zone conductrice est mise à un potentiel nul.

L'invention est adaptée pour mettre en œuvre le principe d'électrosmose de type fréquentiel, consistant à appliquer un signal électrique (tension ou courant) non continu dans le circuit. L'utilisation d'un courant électrique fréquentiel (non continu) permet notamment de minimiser les possibles réactions électrochimiques associées au champ électrique appliqué au liquide traité.

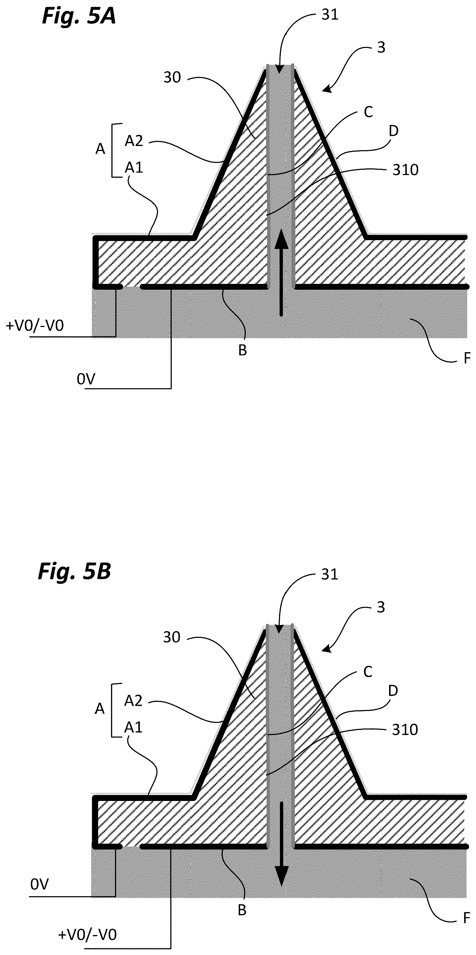

Il faut noter que :
- Selon la polarisation (potentiel nul et potentiel positif ou négatif) appliquée à chaque zone conductrice, on peut piloter le sens de l'écoulement (sens de délivrance de fluide ou sens d'extraction de fluide, voir figures 5A et 5B) : ainsi les polarisations indiquées sur les figures 1, 3, 5A et 5B peuvent être inversées (0V côté zone conductrice A et +V0/-V0 côté zone conductrice B) ;
- Selon l'amplitude du signal électrique imposé entre les deux zones conductrices, on vient piloter le débit de l'écoulement de fluide ;

En outre, il faut également noter que :
- Le débit du fluide lors de l'écoulement dans le canal 31 dépendra de la section de ce canal ;
- Le débit du fluide dépendra de la hauteur du canal 31 : Plus le canal est long, plus le débit est faible à une tension donnée ;
- Le débit du fluide dépendra de sa viscosité : plus le fluide est visqueux, plus le débit est faible ;

L'une des contraintes principales lorsque l'on souhaite réaliser un dispositif tel que celui de l'invention réside dans la reprise de contact sur les zones conductrices.

Comme indiqué ci-dessus, les deux zones conductrices A, B sont agencées majoritairement sur la face interne 20 et sur la face externe 21 du support 2. La reprise de contact peut ainsi être réalisée sur les deux faces opposées du support des microaiguilles afin de polariser le canal avec, par exemple, un potentiel alternatif d'amplitude +V0/-V0 d'un côté et le potentiel 0V de l'autre. Cependant, une variante avantageuse (représentée sur la figure 3) consiste à prolonger la première zone conductrice A sur la face externe du support, en passant par la tranche et en maintenant une zone isolante E du côté de la face externe entre la première zone conductrice A et la deuxième zone conductrice B.

Etant donné que le support peut porter plusieurs micro-aiguilles alignées, les différentes configurations évoquées ci-dessus s'appliquent à toutes les micro-aiguilles d'un même support. On vient ainsi réaliser uniquement deux reprises de contact (par exemple sur la face interne et sur la face externe ou uniquement sur la face externe) sur le support 2 pour l'ensemble des microaiguilles 3, et tous les canaux sont alors polarisés de manière identique.

Selon un autre aspect pertinent de l'invention, le signal électrique imposé entre les deux zones conductrices A, B est avantageusement choisi non continu. Le choix d'appliquer un signal de courant alternatif permet de minimiser les potentielles réactions électrochimiques parasites (électrolyse par exemple). C'est le changement de potentiel d'une part sur la face interne ou sur la face externe du corps qui permettra d'inverser le sens d'écoulement du fluide à travers le canal. La figure 5A montre un écoulement dans le sens de l'extraction de fluide F à travers le canal 31 de la microaiguille 3 et la figure 5B montre un écoulement dans le sens de la délivrance de fluide F à travers le canal 31 de la microaiguille.

Il faut noter également qu'un compromis doit être trouvé pour générer des forces électroosmotiques suffisantes tout en maintenant un échauffement par effet Joule acceptable.

L'utilisation d'un principe d'électro-osmose fréquentielle (signal électrique appliqué non continu) permet de minimiser les possibles réactions électrochimiques associées au champ électrique appliqué au fluide.

## Revendications

1. Dispositif adapté pour être employé dans un système de délivrance/extraction de fluide à travers la peau d'un être vivant, ledit dispositif comprenant :
- Un support (2) comportant deux faces opposées, dites face interne (20) et face externe (21), sa face interne (20) comportant N microaiguilles, N étant supérieur ou égal à 1, chaque microaiguille (3) comportant un corps (30) dans lequel est réalisé un canal (31) de délivrance/extraction de fluide (F), ce canal (31) comportant une paroi interne (310) le long de laquelle le fluide est apte à s'écouler lors de sa délivrance ou de son extraction, **Caractérisé en ce que** :
- Le dispositif comporte une première zone conductrice (A) réalisée sur la face interne (20) du support et une deuxième zone conductrice (B) réalisée sur la face externe (21) du support et **en ce que**, pour chaque microaiguille :
- Une troisième zone conductrice (C) est réalisée sur la paroi interne (310) de son canal (31), cette troisième zone conductrice (C) reliant la première zone conductrice (A) à la deuxième zone conductrice (B), et **en ce que** la troisième zone conductrice (C) présente une conductivité électrique inférieure à celle de la première zone conductrice et de la deuxième zone conductrice.

2. Dispositif selon la revendication 1, **caractérisé en ce que** la première zone conductrice (A) et la deuxième zone conductrice (B) sont chacune réalisées respectivement par un premier dépôt conducteur et un deuxième dépôt conducteur.

3. Dispositif selon la revendication 2, **caractérisé en ce que** le premier dépôt conducteur et le deuxième dépôt conducteur sont métalliques.

4. Dispositif selon la revendication 2 ou 3, **caractérisé en ce que** la troisième zone conductrice (C) est réalisée par un troisième dépôt conducteur.

5. Dispositif selon la revendication 4, **caractérisé en ce que** le troisième dépôt conducteur est réalisé sous la forme d'un polymère conducteur, ou d'un revêtement de type DLC.

6. Dispositif selon l'une des revendications 1 à 5, **caractérisé en ce que** la première zone conductrice (A) se prolonge sur la face externe du support, en transitant par la tranche du support (2).

7. Dispositif selon l'une des revendications 1 à 6, **caractérisé en ce qu'**il comporte un dépôt (D) d'un matériau isolant et biocompatible réalisé sur la première zone conductrice (A).

8. Dispositif selon l'une des revendications 1 à 7, **caractérisé en ce que** le corps (30) de chaque micro-aiguille (3) est réalisé dans un matériau choisi parmi le PLA (acide polylactique), le PLGA (acide polylactique-co-glycolique) et le verre.

9. Dispositif selon la revendication 8, **caractérisé en ce que** le corps (30) de chaque micro-aiguille et le support (2) sont réalisés dans un même matériau.

10. Système de délivrance/extraction de fluide à travers la peau d'un être vivant, comprenant :
- Un dispositif destiné à être appliqué à travers la peau de l'être vivant,
- Une source d'alimentation électrique (S) apte à imposer un signal électrique non continu,
**Caractérisé en ce que** le dispositif est tel que défini dans l'une des revendications 1 à 9, et **en ce que** la source d'alimentation électrique est connectée à sa première zone conductrice et à sa deuxième zone conductrice.

11. Système selon la revendication 10, **caractérisé en ce que** la source d'alimentation électrique est contrôlée pour imposer un signal électrique non continu.

12. Système selon l'une des revendications 10 ou 11, **caractérisé en ce que** le signal électrique est un signal de tension ou un signal de courant imposé entre les deux zones conductrices.

13. Système selon la revendication 12, **caractérisé en ce qu'**il comporte des moyens de réglage du débit de délivrance ou d'extraction du fluide, par ajustement en amplitude du signal électrique.
